(19) Europäisches Patentamt

European Patent Office

Office européen des brevets

(11) Publication number: **0 470 305 A1**

## (12) EUROPEAN PATENT APPLICATION

(21) Application number: **90308654.4**

(22) Date of filing: **07.08.90**

(51) Int. Cl.⁵: **A61F 2/30, A61C 8/00**

(43) Date of publication of application:
**12.02.92 Bulletin 92/07**

(84) Designated Contracting States:
**BE DE FR GB IT NL**

(71) Applicant: **OSTEOTECH, INC.,**
**1151E Shrewsbury Avenue**
**Shrewsbury New Jersey 07701(US)**

(72) Inventor: **Lyle, John W.**
**28 Inlet Terrace**
**Belmar, New Jersey 07719(US)**

(74) Representative: **Ben-Nathan, Laurence Albert et al**
**Urquhart-Dykes & Lord 91 Wimpole Street London W1M 8AH(GB)**

(54) Osteoprosthetic implant.

(57) The anchorage component (13) of an osteoprosthetic implant (10) is coated with an adherent osteogenic composition (30) comprising demineralized bone powder distributed within a biocompatible, resorbable binding agent such as glycolide-lactide copolymer or collagen. Resorption of the binding agent is accompanied by rapid and deep bone in-growth which firmly anchors the prosthesis to the host bone repair site.

Fig. 5

EP 0 470 305 A1

Rank Xerox (UK) Business Services

## BACKGROUND OF THE INVENTION

This invention relates to implantable osteoprosthetic devices and, more particularly, to such prostheses formed as a composite which includes a bone growth-inducing layer or coating.

Many osteoprosthetic devices, e.g., those used in the reconstruction of the hip joint, are joined to the skeletal system by impacting an anchorage component within the intramedullary canal of the bone or by mechanically fixing the device by means of a bone cement such as a polymethylmethacrylate. However, these methods are not entirely satisfactory due to the tendency of the devices to loosen upon impact or as a result of use over a long period of time.

Tissue ingrowth has also been used in attempts to anchor osteoprosthetic devices in place. In this method, the prosthesis is provided with a porous surface which is intended to foster bone ingrowth and serve as an attachment site for the new bone tissue. The tissue ingrowth approach to anchoring a prosthesis has been adopted for a variety of endoprosthetic devices.

U.S. Patent No. 3,986,212 describes a porous polymeric coating for bone fixation by tissue ingrowth. The porous polymeric materials which are indicated to be useful are those having a specified density and interconnected pores of a specific average pore diameter. Among the polymeric materials disclosed are high density polyethylene and polypropylene or mixtures thereof having certain critical parameters. It is also indicated that the coatings can be mechanically interlocked or chemically bonded to the device.

Similarly, in U.S. Patent No. 4,164,794, an osteoprosthetic device is coated with a porous thermoplastic material of particular properties which is said to be compatible with, and conducive for, the ingrowth of cancellous and cortical bone specules.

The anchorage component of the endoprosthetic device described in U.S. Patent No. 4,202,055 possesses a non-porous polymeric coating in which particles of ceramic have been incorporated. Upon resorption of the ceramic particles, a polymer structure with continuous pores is formed which is penetrated by newly formed bone.

In accordance with U.S. Patent No. 4,713,076, the anchorage component of an osteoprosthetic device possesses a completely resorbable coating which is said to enable fast and deep ingrowth of new bone tissue and anchor the implant within the bone. The coating composition is made up of a calcium compound, e.g., tricalcium phosphate or apatite (hydroxyl apatite) provided in the form of highly porous spherical particles which are embedded in a resorbable, biologically compatible binding agent such as a polyamino acid, polylactate, poly-glycolate, co-condensates of these substances, gelatin or collagen.

Other types of coated osteoprosthetic devices are described in U.S. Patent Nos. 3,808,606; 4,159,358; 4,168,326; 4,35,069; 4,365,356; 4,491,987; 4,652,459; 4,702,930; and, 4,705,694.

The use of pulverised exogenous bone growth material, e.g., derived from demineralized allogenic or xenogenic bone, is also known. See, in this regard, the disclosures of U.S. Patent Nos. 4,485,097; 4,678,470 and 4,743,259; Bolander et al., "The Use of Demineralized Bone Matrix in the Repair of Segmental Defects", The Journal of Bone and Joint Surgery, Vol. 68-A, No. 8, pp. 1264-1273; Glowacki et al., "Demineralized Bone Implants", Symposium on Horizons in Plastic Surgery, Vol. 12, No. 2, pp. 233-241 (1985); Gepstein et al., "Bridging Large Defects in Bone by Demineralized Bone Matrix in the Form of a Powder", The Journal of Bone and Joint Surgery, Vol. 69-A, No. 7, pp. 984-991 (1987); [Mellonig], "Decalcified Freeze-Dried Bone Allograft as an Implant Material in Human Periodontal Defects", The International Journal of Periodontics and Restorative Dentistry, pp. 41-55 (June, 1984); and, Kaban et al., "Treatment of Jaw Defects with Demineralized Bone Implants", Journal of Oral and Maxillofacial Surgery. However, there is no suggestion in any of these prior disclosures of combining an osteoprosthetic component with a bone growth-inducing component based on a powdered bone material.

## SUMMARY OF THE INVENTION

It is an object of the invention to provide an osteoprosthetic implant possessing a bone growth-inducing coating or layer on the anchorage component thereof.

It is a particular object of the invention to provide an endoprosthesis, e.g., a hip joint replacement, in which the anchorage component is coated with an osteogenic coating or layer of demineralized bone powder distributed within a biocompatible, resorbable binding agent.

In keeping with these and other objects of the invention, an osteoprosthetic implant possessing a bone tissue-contacting anchorage component is provided with an adherent bone growth-inducing coating or layer of demineralized bone powder distributed within a biocompatible, resorbable binding agent applied to at least a portion of the surface of the anchorage.

As the binding agent undergoes gradual resorption, the void space which is thus created becomes occupied by new bone ingrowth which results in a firm union between the prosthesis and the pre-existing bone tissue. Unlike the non-osteogenic particles contained in known prosthetic coatings, e.g., those disclosed in U.S. Patent No. 4,202,055 discussed supra, the demineralized bone powder which is present in the endoprosthetic coatings of this invention provides a significant osteogenic effect which accelerates new bone ingrowth.

BRIEF DESCRIPTION OF THE DRAWINGS

In the accompanying drawings in which like numerals refer to like elements:
Figs. 1 to 3 are schematic illustrations in side profile view of various stages of one method of applying a bone growth-inducing coating to the anchorage of the femoral component of a hip joint endoprosthesis;
Fig. 4 shows an enlarged section of the bone growth-inducing coating as applied to the hip joint endoprosthesis of Fig. 3; and,
Fig. 5 shows the insertion of the coated hip joint endoprosthesis of Fig. 4 in place within the intramedullary canal of the femur.

DESCRIPTION OF THE PREFERRED EMBODIMENTS

The demineralized pulverized or powdered bone which is incorporated in the osteogenic layer or coating herein is a known type of material and is prepared in accordance with known procedures.

In a preferred bone demineralization procedure, the bone is first pulverized to the desired average particle size followed by defatting/disinfecting and acid demineralization treatments. A preferred defatting/disinfectant solution is an agueous solution of ethanol, the ethanol being a good solvent for lipids and the water being a good hydrophilic carrier to enable the solution to penetrate more deeply into the bone. The agueous ethanol solution also disinfects the bone by killing vegetative microorganisms and viruses. Ordinarily at least about 10% to 40% water (i.e., about 60% to 90% defatting agent such as alcohol) should be present in the defatting, disinfecting solution to produce optimal lipid removal and disinfection within the shortest period of time. The preferred concentration range of the defatting solution is about 60% to 85% alcohol and most preferably 70% alcohol. Following defatting, the bone undergoes a pH controlled immersion in an acid, such as 0.6N hydrochloric acid, for about 3 hours to effect demineralization. Other acids which can be employed in the same or other concentrations include other inorganic as well as organic acids such as peracetic acid. After acid treatment, the bone powder is rinsed with water for injection, buffered with a buffering agent such as 0.1M sodium phosphate solution to a final controlled pH and then finally rinsed with water for injection to remove residual amounts of hydrochloric acid and sodium phosphate. The demineralized bone powder can be used immediately for the preparation of the coated osteoprosthetic device of this invention or it can be stored under aseptic conditions, advantageously in a freeze-dried state, prior to use herein.

The size of the bone particles can vary widely, with average particle sizes of from about 100 microns to about 20 millimeters and preferably from about 200 microns to about 15 millimeters being generally suitable in most cases. The amount of bone powder which can be incorporated into the binding agent to provide the osteogenic layer or coating of this invention can also vary widely with amounts of from about 5 to about 80 weight percent, and preferably from about 20 to about 60 weight percent, being entirely suitable in most cases.

If desired, the bone powder can be modified in one or more ways, e.g., the porosity of the bone powder can be increased and/or the bone powder can be treated with one or more modifying agents, e.g., glutaraldehyde, as disclosed in U.S. Patent No. 4,678,470. Another optional treatment involves the augmentation of the bone protein content of the powdered bone employing the procedure of U.S. Patent No. 4,743,259. Prior to incorporation within the binding agent, any of a variety of substances can be introduced into the bone particles, e.g., by soaking or immersing the bone particles in a solution of the desired substance(s) followed by drying of the bone particles. Substances which can be readily incorporated in the bone particles in this or any other suitable manner include antiviral drugs, e.g., those suitable for preventing transmission of acquired immune deficiency syndrome (AIDS); antimicrobials and/or antibiotics such as erythromycin, bacitracin, neomycin, penicillin, polymyxin B, tetracyclines, viomycin, chloromycetin and streptomycins, cefazolin, ampicillin, tobramycin, clindamycin and gentamicin, etc.; amino acids, peptides, vitamins, inorganic elements, NAD and/or other nutrients; hormones; endocrine tissue or tissue fragments; synthesizers; enzymes such as collagenase, peptidases, oxidases, etc.; polymer-cell scaffolds with parenchymal cells; angiogenic drugs and polymeric carriers containing such drugs; collagen lattices; biocompatible surface active agents; antigenic agents; cytoskeletal agents; biologically active components such as bone morphogenetic proteins (BMPs), transforming growth factor (TCF-beta), insulin-like growth factor (IGD-1); mesenchymal elements; bone digestors; antitumor agents; cellular attractants and attachment agents; immunosuppressants; and, nucleic acids. In addition to adding such substances to the demineralized bone powder or as an alternative to such addition, these and other substances can also be introduced into the binding agent component of the osteogenic coating or layer. The amounts of optionally added substances can vary widely with optimum levels being readily determined in a specific case by routine experimentation.

A variety of biocompatible and resorbable, i.e., biodegradable or bioerodable, materials are available for use as the binding agent component of the osteogenic layer or coating herein. Among such materials which can advantageously be used are polyglycolic acid, polylactic acid, glycolide-lactide copolymers and modifications of such copolymers with one or more other copolymerizable monomers such as e-caprolactone, polyethylene oxide-polyethylene terephthalate copolymers, the polyesteramides disclosed in U.S. Patent No. 4,826,945, the polyanhydrides disclosed in Rosen et al., Biomaterials, 4, 131 (1983) and Leong et al., J. Biomed. Mater. Res., 19 (8), 941 (1985), the polyether glycol-based multiblock copolymers disclosed in U.S. Patent No. 4,826,945, bioerodable cyanoacrylates, the orthoester polymers and orthocarbonate polymers disclosed in U.S. Patent No. 4,180,646, uncrosslinked and crosslinked collagen and chitin. For a description of a demineralized bone powder-containing collagen which can be applied to an osteoprosthetic implant in accordance with this invention, reference may be made to U.S. Patent No. 4,485,097, the contents of which are incorporated by reference herein. If desired, the binding agent can contain a reinforcement agent, e.g., a fibrous or particulate reinforcement agent, to increase the tensile strength of the osteogenic coating.

Although the invention will now be illustrated in connection with a hip joint endoprosthesis, it is to be understood that the invention can be practiced with any type of bone implant or replacement, e.g., one used in dental or maxillofacial reconstruction.

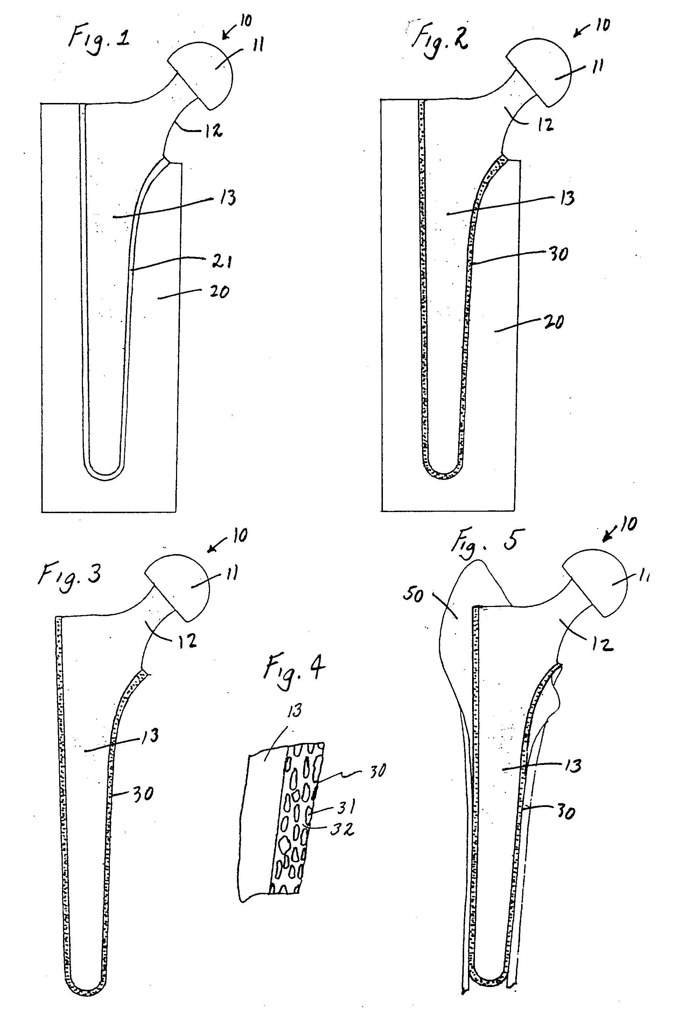

As shown in Figs. 1-5, femoral component 10 of a known type of hip joint prosthesis is fabricated from any of a variety of bioengineering materials such as metal, ceramic, polymers and their composites, and the like. The prosthesis includes a head 11, a neck 12 and a stem 13 which serves as an anchorage to secure the implant within the intramedullary canal of femur 50. In the method of applying osteogenic layer 30 illustrated in Figs. 1 to 3, femoral component 10 is placed within the cavity of mold half 20 (the other matching mold half is not shown) with space or gap 21 defined by the surface of stem 13 and the wall of the mold cavity corresponding to the shape and thickness of osteogenic layer 30 to be applied to the stem.

In one convenient method of applying the osteogenic coating, a plasticized mass of binding agent is first prepared with the demineralized bone being substantially uniformly incorporated therein. Thus, for example, where the binding agent is a glycolide-lactide copolymer, a quantity of the resin is plasticized to a paste-like consistency utilizing a suitable solvent such as benzene, toluene, xylene, and the like, followed by uniform incorporation of the bone powder therein. The fluent mass of material can be applied to the surface of stem 13 in excess and following placement of the prosthesis in the mold and closing of the mold, excess material is expelled from the mold. Alternatively, the uncoated prosthesis is positioned within mold half 20, the other mold half is locked in place and a quantity of the plasticized osteogenic coating material is injected just to the point of excess within space 21. Following formation of osteogenic layer 30 about stem 13 as shown in Fig. 2, the prosthesis is removed from the mold and, following evaporation of the plasticizing solvent, the coated prosthesis, shown in Fig. 3, is now ready for sterilization and aseptic packaging.

In yet another technique for applying osteogenic coating 30 to the anchorage component of endoprosthesis 10, the binding agent is provided as a fine powder, e.g., of an average particle size approximately that of the demineralized bone powder, the latter being uniformly mixed with the binding agent powder to provide a dry, readily flowing powder mixture which is then introduced to excess within space 21 of mold 20. Thereafter, heating of the powder mixture to a temperature at which it forms a self-supporting adherent coating, e.g., a temperature which is sufficient to weld or melt the binding agent particles into a unitary mass, followed by cooling to ambient temperature provides the coated prosthetic device of this invention.

Osteogenic coating 30 can also be applied to the surface of stem 13 by first applying a solvent solution of binding agent to the stem and, following partial evaporation of the solvent to provide a tacky coating, demineralized bone powder is contacted with, and retained by, the binding agent. This procedure can be repeated several times to build the coating up to a predetermined thickness. Complete evaporation of solvent results in the fully formed osteogenic coating.

Still another procedure which is especially suitable for applying an osteogenic layer based on soluble collagen as the binding agent involves immersing the prosthesis in an aqueous collagen gel containing suspended demineralized bone powder followed by drying of the gel and, if desired, crosslinking the collagen binder to any desired degree utilizing a crosslinking agent such as formaldehyde in accordance with known procedures.

The thickness of the osteogenic coating or layer is not especially critical. Average thicknesses of from about 1 to about 50 mils and advantageously from about 10 to about 40 mils generally provide satisfactory results.

If desired, the surface of the prosthesis, e.g., stem 13, which is to receive the osteogenic coating or layer can be subjected to one or more preparative treatments in order to enhance the adhesion of the osteogenic coating or layer thereto. For example, the surface of the prosthesis can be provided with an adhesion-promoting pattern formed as described in U.S. Patent No. 4,778,469, or an adhesion-promoting roughened surface texture as described in U.S. Patent No. 4,159,358.

The following example is illustrative of the prosthetic implant and osteogenic coating composition of this invention.

EXAMPLE

A. Preparation of Demineralized Cortical Bone Powder

A quantity of cortical bone which has been pulverized and sieved to an average particle size of from about 100 to about 300 microns is introduced into a reactor which is then sealed. A 70% ethanol solution at the rate of 30 milliliters per gram of bone is introduced into the reactor followed by agitation for 1 hour (Bolander et al., Journal of Bone and Joint Surgery, Vol. 68-A, No. 8 (Oct. 1986)) to effect defatting and disinfecting of the bone powder. Following drainage of the ethanol, a 0.6N solution of HCl at a rate of 50 ml per gram of bone is introduced into the reactor (Bolander et al., ibid.), the reaction proceeding for 3 hours (Glowackie, AATB Workshop, 11th Annual meeting (1987)). Following drainage of the HCl, the bone is covered and rinsed three times with water for injection (WFI) with the WFI being replaced at 5 minute intervals. Following drainage of the WFI, the bone is completely covered with 0.1M sodium phosphate, a procedure which is repeated until the pH of the solution falls between 6.8 and 7.4. The rinsing procedure with WFI is repeated to provide demineralized cortical bone powder ready for application herein.

B. Application of Demineralized Cortical Bone Powder to The Stem of a Hip Joint Endoprosthesis

A binding agent solution of glycolide-lactide copolymer in xylene is prepared by dissolving 20 weight percent glycolide-lactide copolymer (approximately 20 mole percent glycolide) in the form of a powder in 80 weight percent xylene. The stem of a hip joint prosthesis is dipped in the binding agent solution, the solution is dried to a tacky consistency and bone powder is dusted onto the surface of the binding agent to which it readily adheres. The procedure is repeated several times to build up an osteogenic layer of about 2-3 millimeters average thickness and containing from about 40 to about 50 weight percent demineralized cortical bone powder on the surface of the stem of the prosthesis. Following complete evaporation of solvent in a drying chamber, the coated prosthesis is sterilized and packaged employing known and conventional procedures.

## Claims

1. An osteoprosthetic device, the anchorage component of which possesses an adherent coating of an osteogenic composition comprising demineralized bone powder distributed within a biocompatible, resorbable binding agent.

2. The osteoprosthetic device of Claim 1 wherein the average particle size of the demineralized bone powder is from about 100 microns to about 20 millimeters.

3. The osteoprosthetic device of Claim 1 wherein the average particle size of the demineralized bone powder is from about 200 to about 15 millimeters.

4. The osteoprosthetic device of Claim 1 wherein the osteogenic coating contains from about 5 to about 80 weight percent demineralised bone powder.

5. The osteoprosthetic device of Claim 1 wherein the osteogenic coating contains from about 20 to about 60 weight percent demineralized bone powder.

6. The osteoprosthetic device of Claim 1 wherein the demineralized bone powder is derived from cortical bone.

7. The osteoprosthetic device of Claim 1 wherein the binding agent is selected from the group consisting of polyglycolic acid, polylactic acid, glycolide-lactide copolymer, glycolide-lactide copolymer modified with one or more monomers copolymerizable therewith, polyethylene oxide-polyethylene terephthalate copolymer, polyesteramide, polyanhydride, polyether glycol-based multiblock copolymer, bioerodable cyanoacrylate, orthoester polymer, orthocarbonate polymer, uncrosslinked and crosslinked collagen and chitin.

8. The osteoprosthetic device of Claim 1 wherein the osteogenic layer or coating contains at least one additional ingredient selected from the group consisting of reinforcing fiber and reinforcing particle.

9. The osteoprosthetic device of Claim 1 wherein the osteogenic layer or coating contains at least one additional ingredient selected from the group consisting of antiviral agent, antimicrobial agent, antibiotic agent, amino acid, peptide, vitamin, inorganic element, NAD, hormone, endocrine tissue, synthesizer, enzyme, polymer-cell scaffolding agent with parenchymal, angiogenic drug, polymeric drug carrier, collagen lattice, antigenic agent, cytoskeletal agent, biologically active component, mesenchymal agent, bone digestor, antitumor agent, cellular attractant, cellular attachment agent, immunosuppressant and nucleic acid added to the bone particles, the binding agent or both.

10. The osteoprosthetic device of Claim 1 in which the thickness of the osteogenic layer is from about 1 to about 50 mils.

11. The osteoprosthetic device of Claim 1 in which the thickness of the osteogenic layer is from about 10 to about 40 mils.

12. The osteoprosthetic device of Claim 1 in which the anchorage component has been provided with an adhesion-promoting surface prior to application of the osteogenic composition thereto.

13. The osteoprosthetic device of Claim 1 wherein the device is an orthopedic, dental or maxillofacial prosthesis.

Fig. 1

Fig. 2

Fig. 3

Fig. 4

Fig. 5

European
Patent Office

**EUROPEAN SEARCH
REPORT**

Application Number

**EP 90 30 8654**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-3 892 648   (PHILLIPS et al.)<br>* column 1, line 56 - column 2, line 5; claim 3 *<br>– – – | 1,7 | A 61 F 2/30<br>A 61 C 8/00 |
| D,Y | US-A-4 485 097   (BELL)<br>* claim 1; column 2, lines 3-11 *<br>– – – | 1,7 | |
| A | EP-A-0 169 001   (COLLAGEN CORP.)<br>* claims 1,4; page 9, line 17 - page 10, line 11 *<br>– – – | 1,7 | |
| A | US-A-4 051 598   (SNEER)<br>* claim 1 *<br>– – – | 1,13 | |
| A | US-A-3 918 100   (SHAW et al.)<br>* claims 1; column 5, lines 40-56 *<br>– – – | 1 | |
| D,A | US-A-4 202 055   (REINER et al.)<br>* claims 1-3 *<br>– – – – – | 1,7,13 | |

TECHNICAL FIELDS
SEARCHED (Int. Cl.5)

A 61 C
A 61 F
A 61 L

The present search report has been drawn up for all claims

| Place of search | Date of completion of search | Examiner |
|---|---|---|
| Berlin | 08 April 91 | KANAL P K |

CATEGORY OF CITED DOCUMENTS
X : particularly relevant if taken alone
Y : particularly relevant if combined with another
    document of the same catagory
A : technological background
O : non-written disclosure
P : intermediate document
T : theory or principle underlying the invention

E : earlier patent document, but published on, or after
    the filing date
D : document cited in the application
L : document cited for other reasons
--------------------------------------------------------------------
& : member of the same patent family, corresponding
    document